(19) Europäisches Patentamt
European Patent Office
Office européen des brevets

(11) **EP 4 702 920 A1**

(12) **EUROPEAN PATENT APPLICATION**

(43) Date of publication:
**04.03.2026 Bulletin 2026/10**

(51) International Patent Classification (IPC):
***A61B 5/11*** (2006.01)     ***A61B 5/00*** (2006.01)

(21) Application number: **24196342.0**

(22) Date of filing: **26.08.2024**

(52) Cooperative Patent Classification (CPC):
**A61B 5/686; A61B 5/1116; A61N 1/36542;**
A61B 5/0031; A61B 2562/0219; A61N 1/36514

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **BIOTRONIK SE & Co. KG**
**12359 Berlin (DE)**

(72) Inventor: **HUEGERICH, Burkhard**
**Portland, 97202 (US)**

(74) Representative: **Biotronik Corporate Services SE**
**Corporate Intellectual Property**
**Sieversufer 9**
**12359 Berlin (DE)**

(54) **BODY'S POSTURE DETERMINATION FROM ACCELEROMETER DATA OF A NOT GRAVITY FORCE DIRECTION ALIGNED IMPLANTED DEVICE**

(57) A method (300) for determining a posture of a patient comprises obtaining at least one angular value representing acceleration data of the patient, obtaining posture data comprising at least one angular range representing at least one predetermined posture, and determining the posture of the patient (360) based at least partly on the at least one angular range and the angular value.

300

FIG. 3A

EP 4 702 920 A1

**Description**

The present invention relates to methods, apparatuses, and computer programs for determining a posture of a patient.

Acceleration sensors are widely used to determine the posture of a patient equipped with said acceleration sensor. E.g., active electronic implantable medical devices may often comprise such sensor to determine the patient's posture. However, it is often difficult to align the sensor with the body it is mounted to. Light inaccuracies of aligning the sensor and the body will falsify all orientations determined by said sensor unless one accounts for the orientation of the sensor relative to the body.

Especially in medical applications, e.g., comprising implanting the sensor into the patient's body, it may even be impossible to align the sensor with the patient's body without risking the patient's health. Thus, the orientation of the sensor in the patient after mounting the sensor to the body, e.g., by implantation, must be known. Typically, this is done via post-mounting calibration. This may, e.g., in the example of implantable medical devices be based on X-ray images or information on the exact spatial orientation of the implant in the body after implantation. These, however, are usually not available or can only be obtained at great expense. In the example of X-ray, there is even a health risk associated with said calibration. Even if they are available, the information on the orientation of the implant is usually inaccurate.

Hence, there is a need to improve methods, apparatuses, and computer programs for determining patient postures.

The above need is met at least in part by the aspects of the present invention.

According to a first aspect of the present invention, a method for determining a posture of a patient, the method comprises obtaining at least one angular value representing acceleration data of the patient; obtaining posture data comprising at least one angular range representing at least one predetermined posture; and determining the posture of the patient based at least partly on the at least one angular range and the angular value.

The inventor found out that this method combines a plurality of advantages:

Firstly, it may be based on a simple calibration in which the patient may be placed, e.g., in the predetermined posture of interest. Therefore, no precise patient orientation and/or imaging to detect the orientation of the means that may provide the acceleration data (e.g., an implanted acceleration sensor) is required. Implantable medical devices serve a multitude of information needs regarding the overall health of patients. Determining, e.g., the upright or supine position of the patient at any given time adds to the better evaluation of the current or developing status of the patient. Determining the posture of the patient (e.g., during nighttime) and its change over time can help identifying underlying health issues which causes, e.g., unusual nighttime postures of the patient. Analogously, e.g., determining the daily amount of time spent in an upright posture might help guiding a patient's path to recovery.

Secondly, this method works completely independent from the orientation and position of the means which provide the acceleration data. In more detail, the invention allows to determine the posture of a patient's body (e.g., between upright and supine) without the need of knowing said exact orientation and position within the patient's body. This allows the attending health professional to implant the means that may provide the acceleration data (e.g., comprised in an implantable medical device) exclusively according to the medical requirements which may increase patient safety and treatment success while reducing unwanted side effects and/or decreased implantable medical device performance in terms of its dedicated function.

Thirdly, the use of angular value representing acceleration data of the patient was shown to be, on the one hand, well-handleable for users, e.g., in order to adjust the angular ranges described herein in an intuitive fashion which may increase user satisfaction and reduce potential errors that might arise from unintuitive alternatives. On the other hand, angular values, as they account for the geometric rotations that may occur in space upon posture changes may reduce the required computation capability requirements and thus energy consumption of any device that may execute at least a part of the steps of the method. This may be particularly advantageous when an implantable medical device executes at least a part of the method as described herein as typically, their battery lifetime is desired to be as long as possible.

The steps of the method described herein may be performed by one or multiple devices: In one example, a remote server may obtain the at least one angular value representing acceleration data of the patient upon them being transmitted to said server, e.g., from an implantable medical device that may have measured said acceleration data. The server may further obtain posture data comprising at least one angular range representing at least one predetermined posture, e.g., also from the implantable medical device that may have measured said calibration data and/or from a database. In such database, e.g., the at least one angular range may be stored, e.g., after having been determined based on the calibration data. Generally, the step of determining the angular range may be performed by the server, the implantable medical device, and/or a further device, e.g., a device coupled to the database. Further, the server may be configured to determine the posture of the patient based at least partly on the at least one angular range and the angular value. Optionally, it may provide, e.g., via (wireless) transmission, the determined posture and/or instructions based on the determined posture to the implantable medical device associated with the patient and/or the posture. Said instructions may, e.g., instruct the device to change at least one operation parameter in response to the determined posture. E.g., a pain-relieving stimulation of a spinal cord stimulator may be reduced in amplitude and/or frequency when the patient is in a supine posture compared to a situation wherein the patient is in an upright posture.

In another examples, at least one or all of the steps of the method may be executed by the implantable medical device and/or a remote device (e.g., a mobile device, mobile phone, smart watch, ...) coupled to the implantable medical device.

In an exemplary embodiment of the present invention, the obtaining the at least one angular value may comprise measuring the acceleration data of the patient. Preferably, the obtaining the at least one angular value may further comprise transforming the acceleration data into the at least one angular value. Optionally, the transforming the acceleration data may be based on a look-up table.

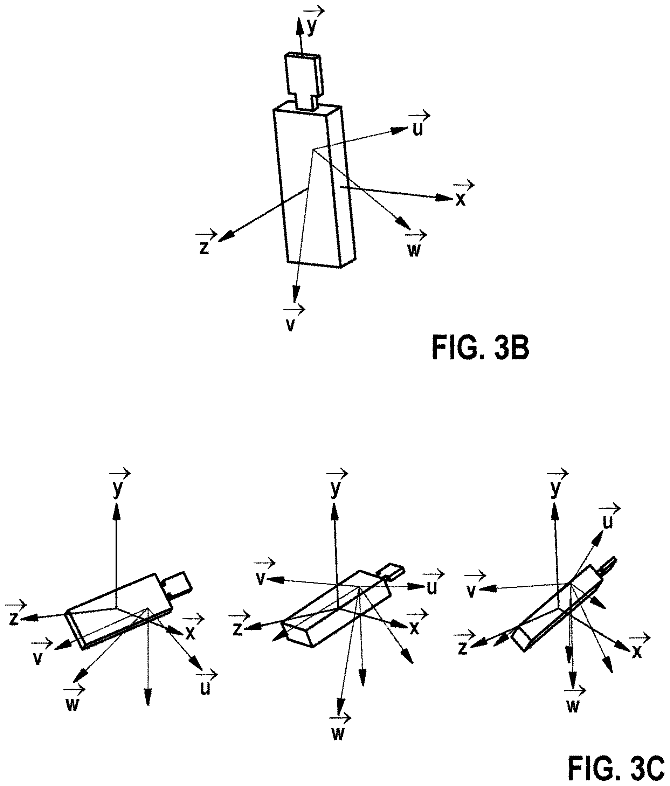

The inventor found that angular values are particular useful to represent posture (changes) as these directly translate to angular rotations about different axes through the patient's body (e.g., as shown schematically in Figs. 3b and 3c). Transforming based on a look-up table may significantly reduce the required computing capabilities and thus the energy consumption of the means executing the transforming. This allows, e.g., to execute this (and/or all other steps) by an implantable medical device, e.g., as described herein, wherein energy consumption and computing power is typically a central issue due to the difficulties of battery exchange requiring surgery and the potential size limitation of the implantable device.

Obtaining the at least one angular value may, e.g., comprise measuring said angular value, determining it based on another value (e.g., as described herein) and/or receiving it from another means configured to provide said at least one angular value.

The angular value may, e.g., comprise an angle (e.g., expressed in degrees (°) and/or multiples of $\pi$) and/or any representation thereof. For example, an acceleration sensor may provide acceleration data along one axis, e.g., an x-axis: The acceleration data $a_x$ in x-direction may comprise any value from -g to +g, wherein $g = |\vec{g}|$ is the magnitude of the gravity vector $\vec{g}$. Accordingly, the value $a_x$ may be understood as the angular value or the angle derived therefrom, e.g., the angle relative to the $\vec{g}$-axis $\phi_x = \arccos(a_x/g)$ may be understood as the angular value. The same applies to any other analogous representation of $a_x$ and/or $\phi_x$. The same may, e.g., be executed analogously for further axes (e.g., a y-axis and/or a z-axis) as described herein.

In some examples, the obtaining the posture data may comprise obtaining, preferably measuring, calibration data with the patient in the at least one predetermined posture. Preferably, the obtaining the posture data may comprise transforming the calibration data into at least one angular calibration value. Optionally, the transforming the calibration data may be based on a look-up table.

The inventor found that working based on angular (calibration) values could provide a particularly suitably parameter space, allowing to address variations of the patient posture in the linear space of the angle observed in degrees rather than the nonlinear space of the cosine of the represented angle.

The obtaining, measuring and/or transforming of the calibration data may, generally be executed as described herein for the acceleration data.

In some examples, the acceleration data may comprise orthogonal data along at least two axes, preferably orthogonal data along three axes. In some examples, the calibration data may comprise orthogonal data along at least two axes, preferably orthogonal data along three axes. Preferably, the acceleration data and the calibration data may comprise data along the same axes.

Thereby, advantageously, the calibration data and the acceleration data may be provided by the same data source, e.g., the same acceleration sensor, e.g., as described herein. Thereby, a logical link between the calibration data and the acceleration data may be ensured, automatically. Having orthogonal axes allows for a mutually independent treatment of the three axes which may simplify the method. Thereby, energy and computational costs may be saved. This may be particularly advantageous when an implantable medical device executes at least a part of the method as described herein as typically, their battery lifetime is desired to be as long as possible.

For example, the acceleration data and/or the calibration data may comprise a vector comprising acceleration values measured by an acceleration sensor along three orthogonal axes.

In an exemplary embodiment of the present invention, the obtaining the posture data may comprise determining the at least one angular range based at least partly on calibration data with the patient in the at least one predetermined posture.

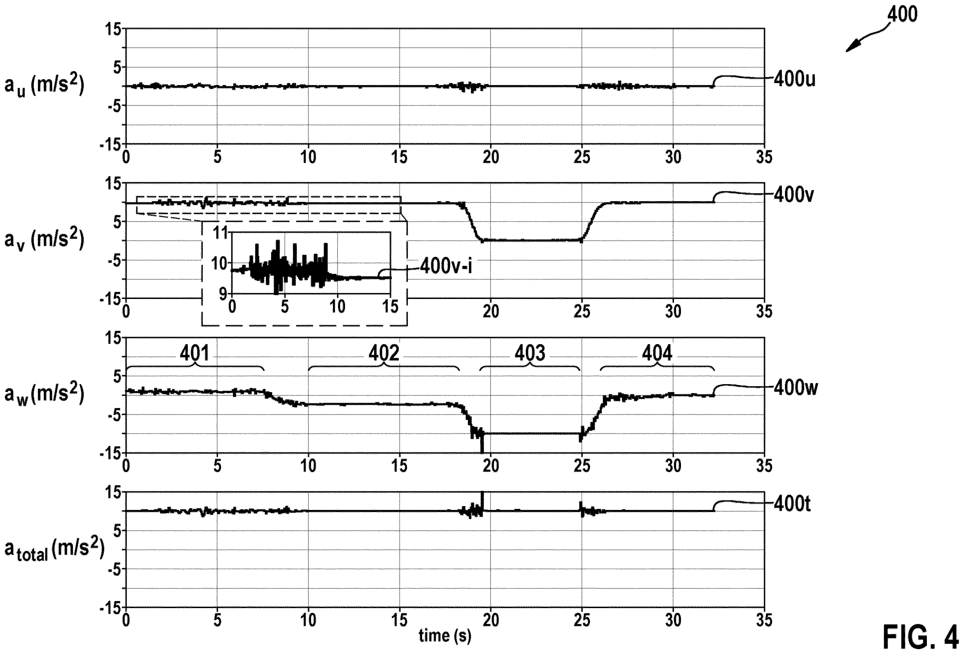

For example, an angular range centered around the calibration data may be determined. E.g., when the calibration data may comprise three-dimensional calibration data that may be represented by and/or transformed into one or more angular calibration values, in this example (90°, 6°, 84°) which corresponds to the initial data of the example of Fig. 4. In this example, a first angular x-range may range from 88° to 92°, a first angular y-range may range from 4° to 8°, and a first angular z-range may range from 82° to 86°.

In some examples, the determining the at least one angular range may be based at least partly on a predetermined deviation, preferably between 0.1% and 10%, more preferably 0.5% to 5%, from the calibration data.

In one example, when the first calibration data comprise an angle of $\phi_{cal,x} = 45°$, and a deviation of 5% is chosen, the corresponding angular range ranges from

$$\phi_{min,x} = 45°(1 - 5\%) = 42.75° \text{ to } \phi_{max,x} = 45°(1 + 5\%) = 47.25°.$$

The inventor could show in first experiments that choosing said predetermined deviation may optimize the accuracy in determining the posture of the patient. In their tests, the method was configured to determine/choose a posture from a predetermined plurality of postures, namely an upright, a supine, a right-tilted, a left-tilted, a prone, a ca. 15° incline, a ca. 30° incline, a ca. 45° incline, and/or an incline posture. Therein, 91% of the determined postures were determined correctly for a deviation of ±1%, 93% for a deviation of ±2%, 93% for a deviation of ±5%, and 92% for a deviation of ±10%.

The inventor found that these ranges result in a particularly reliable determination with optimized (reduced) false-negatives as well as false-positives for the determined postures.

For example, the at least one angular range may be based at least partly on a predetermined deviation angle, preferably between 0.1° and 10°, more preferably 0.5° to 5°, from the calibration data.

In this example, when the first calibration data comprise an angle of $\phi_{cal,x} = 45°$, and a deviation of 5° is chosen, the corresponding angular range ranges from $\phi_{min,x} = 45° - 5° = 40°$ to $\phi_{max,x} = 45° + 5° = 50°$. The same may, e.g., be applied to one or more further axes, e.g., the y- and/or z-axis as described herein. Different deviations or the same deviation may be chosen for a plurality of axes.

In some examples, the calibration data may comprise at least first calibration data for a predetermined first posture of the patient and second calibration data for a predetermined second posture of the patient.

By choosing two postures for calibration, e.g., an upright posture as the first posture and a supine posture as a second posture, the method may be improved in its accuracy and reliability - especially regarding those postures that are of interest. E.g., when distinguishing between said upright and supine posture is desired, using these as predetermined first and second (calibration) postures was shown to enhance the reliability of determining just these postures significantly.

For example, the first and the second posture may vary by 70° to 110°, preferably by 80° to 100°, e.g., a first posture may comprise a body orientation that deviates from a body orientation of the second posture by 70° to 110°, preferably by 80° to 100°. Optionally, the first posture may comprise an upright posture of the patient and/or the second posture of the patient may comprise a supine posture of the patient.

Advantageously, having a certain difference (e.g., expressed by the angles above) between the first and second (calibration) postures can improve the accuracy and reliability of the subsequent steps of the method.

In some examples, the at least one angular range may comprise at least: a first angular range based at least partly on the first calibration data, a second angular range based at least partly on the second calibration data, and preferably a global range representing a posture range comprising at least the first and the second posture.

These ranges, and the determining of the posture based thereon was shown to yield particularly reliable results.

For example, when the first angular range (e.g., for an upright posture) ranges from $\phi_{min,x,upr} = 2°$ to $\phi_{max,x,upr} = 6°$ and the second angular range (e.g., for a supine posture) ranges from $\phi_{min,x,upr} = 92°$ to $\phi_{max,x,upr} = 96°$, the global range representing the posture range from the upright to the supine posture may range from 2° to 96°.

The method may, for example, further comprise obtaining, preferably for each axis, an average value of the first calibration data and the second calibration data. Preferably, the at least one angular range may comprise at least one range around the average value.

The inventor found out that even though such average value does not represent either of the postures used for calibration, it may serve as a suitable reference point that may be used as a further basis for the determining of the patient posture.

For example, when the first angular value (e.g., for an upright posture) is $\phi_{x,upr} = 4°$ and the second angular value (e.g., for a supine posture) is $\phi_{x,upr} = 94°$ the average value of the first calibration data and the second calibration data may be

$$\phi_{x,avg} = \frac{(\phi_{x,upr} - \phi_{x,sup})}{2} = 49°.$$

For example, the range may be defined by all those acceleration data $\phi_x$ that deviate from said $\phi_{x,avg}$ by less than a predetermined average deviation $D_{avg}$, e.g., of a maximum of $D_{avg} = 40\%$, 50% or 60%. The deviation may, e.g., be calculated by $\phi_{\%,off} = (\phi_x - \phi_{x,avg})/\phi_{x,avg}$ such that $\phi_{\%,off} \leq D_{avg}$.

The inventor could show in first experiments that choosing said average deviation $D_{avg}$ may optimize the accuracy in determining the posture of the patient. In his tests, the method was configured to determine/choose a posture from a predetermined plurality of postures, namely an upright, a supine, a right-tilted, a left-tilted, a prone, a ca. 15° incline, a ca. 30° incline, a ca. 45° incline, and/or an incline posture. Therein, a 90% of the determined postures were determined correctly for $D_{avg}$ = 40%, 95% for $D_{avg}$ = 50%, and 91% for $D_{avg}$ = 60%.

In some examples, the determining the posture of the patient may be based at least partly on whether the calibration data lie within the at least one angular range (e.g., in one or more of the ranges described herein).

This poses a particularly reliable yet efficient basis for the determining of the posture described herein.

In some examples, the determining the posture of the patient may comprise choosing a posture from a predetermined plurality of postures.

The plurality of postures may, e.g., comprise an upright, a supine, a right-tilted, a left-tilted, a prone, a ca. 15° incline, a ca. 30° incline, a ca. 45° incline, and/or an incline posture. Postures from this selection proved to be particularly useful for providing the attending health professional and/or remote patient monitoring workflow with sufficiently accurate yet manageable information.

In an exemplary embodiment of the present invention, the acceleration data may be obtained from an implantable sensor implanted into a body of the patient.

Using an implanted sensor may advantageously guarantee that the acceleration data and/or calibration data measured by said sensor are representative for the posture of the patient: In detail, the sensor and the (patient's) body (at least at and near the implantation site) may rotate together but not with respect to one another.

For example, the implantable sensor may be comprised in an implantable medical device, e.g., as described herein.

The method may, for example, further comprise a calibration step, the calibration step comprising measuring the calibration data with the patient in at least one predetermined posture.

By ensuring that the patient is in at least one predetermined (and suitable) posture, the posture determination - which depends on the calibration - may be performed with increased accuracy and/or reliability.

According to a second aspect of the present invention, an implantable medical device comprises: means for obtaining an angular value representing acceleration data of the patient; means for obtaining posture data comprising at least one angular range representing at least one predetermined posture; and means for determining the posture of the patient based at least partly on the at least one angular range and the angular value.

Such implantable medical devices may be optimized in their performance when configured as described herein. For example, implantable medical devices providing therapy to the patient (like, e.g., spinal cord stimulators, pacemakers, etc.) may be optimized in their therapeutic delivery as said delivery may be tailored to the current patient posture, e.g., indicating whether the patient is lying, standing, etc. as such parameters may change the required therapeutic delivery. Equally, the implantable medical device comprising, e.g., a medical sensor may provide physiological data that may be interpreted differently depending on the current patient posture, e.g., indicating whether the patient is lying, standing, etc. Thereby, device performance may be enhanced in various aspects.

Said implantable medical device may, e.g., comprise an (implantable) sensor, e.g., an acceleration sensor comprising the means for obtaining the angular value representing acceleration data of the patient, e.g., as described herein.

It is noted that the aspects described herein with reference to a method may be implemented as functions of a device, e.g., by means of hard and/or software, and vice versa.

In some implementations, the sensor, the implantable medical device, the remote device and/or the (remote) server can include a data processor and/or a storage device. The data processor can, e.g., be configured to execute one or more of the steps of the method as described herein. The storage device can store the data and parameters described herein, e.g., the acceleration data, the at least one angular value, the posture data, the at least one angular range, and/or the look-up table. In some implementations, the implantable medical device, the remote device and/or the (remote) server can include one or more computers that include one or more data processors configured to execute one or more programs that include a plurality of instructions according to the principles described above. Each data processor can include one or more processor cores, and each processor core can include logic circuitry for processing data. For example, a data processor can include an arithmetic and logic unit (ALU), a control unit, and various registers. Each data processor can include cache memory. Each data processor can include a system-on-chip (SoC) that includes multiple processor cores, random access memory, graphics processing units, one or more controllers, and one or more communication modules. Each data processor can include millions or billions of transistors.

The processing of data described in this document, such as, e.g., determining the at least one angular range, determining the posture of the patient based at least partly on the at least one angular range and the angular value, transforming the acceleration data and/or the calibration data, and/or determining the at least one angular range based at least partly on calibration data, can be carried out using one or more computers, which can include one or more data processors for processing data, one or more storage devices for storing data, and/or one or more computer programs including instructions that when executed by the one or more computers cause the one or more computers to carry out the processes. The one or more computers can include one or more input devices, such as a keyboard, a mouse, a touchpad, and/or a voice command input module, and one or more output devices, such as a display, and/or an audio speaker.

In some implementations, the one or more computing devices can include digital electronic circuitry, computer hardware, firmware, software, or any combination of the above. The features related to processing of data can be implemented in a computer program product tangibly embodied in an information carrier, e.g., in a machine-readable storage device, for execution by a programmable processor; and method steps can be performed by a programmable processor executing a program of instructions to perform functions of the described implementations. Alternatively, or in addition, the program instructions can be encoded on a propagated signal that is an artificially generated signal, e.g., a machine-generated electrical, optical, or electromagnetic signal, that is generated to encode information for transmission to suitable receiver apparatus for execution by a programmable processor.

Another aspect of the present invention relates to a computer program comprising instructions for executing the steps of the method as described herein.

A computer program can be written in any form of programming language, including compiled or interpreted languages, and it can be deployed in any form, including as a stand-alone program or as a module, component, subroutine, or other unit suitable for use in a computing environment.

For example, the one or more computers can be configured to be suitable for the execution of a computer program and can include, by way of example, both general and special purpose microprocessors, and any one or more processors of any kind of digital computer. Generally, a processor will receive instructions and data from a read-only storage area or a random access storage area or both. Elements of a computer system include one or more processors for executing instructions and one or more storage area devices for storing instructions and data. Generally, a computer system will also include, or be operatively coupled to receive data from, or transfer data to, or both, one or more machine-readable storage media, such as hard drives, magnetic disks, solid state drives, magneto-optical disks, or optical disks. Machine-readable storage media suitable for embodying computer program instructions and data include various forms of non-volatile storage area, including by way of example, semiconductor storage devices, e.g., EPROM, EEPROM, flash storage devices, and solid state drives; magnetic disks, e.g., internal hard disks or removable disks; magneto-optical disks; and CD-ROM, DVD-ROM, and/or Blu-ray discs.

In some implementations, the processes described above can be implemented using software for execution on one or more mobile computing devices, one or more local computing devices, and/or one or more remote computing devices (which can be, e.g., cloud computing devices). For instance, the software forms procedures in one or more computer programs that execute on one or more programmed or programmable computer systems, either in the mobile computing devices, local computing devices, or remote computing systems (which may be of various architectures such as distributed, client/server, grid, or cloud), each including at least one processor, at least one data storage system (including volatile and non-volatile memory and/or storage elements), at least one wired or wireless input device or port, and at least one wired or wireless output device or port.

In some implementations, the software may be provided on a medium, such as CD-ROM, DVD-ROM, Blu-ray disc, a solid state drive, or a hard drive, readable by a general or special purpose programmable computer or delivered (encoded in a propagated signal) over a network to the computer where it is executed. The functions can be performed on a special purpose computer, or using special-purpose hardware, such as coprocessors. The software can be implemented in a distributed manner in which different parts of the computation specified by the software are performed by different computers. Each such computer program is preferably stored on or downloaded to a storage media or device (e.g., solid state memory or media, or magnetic or optical media) readable by a general or special purpose programmable computer, for configuring and operating the computer when the storage media or device is read by the computer system to perform the procedures described herein. The inventive system can also be considered to be implemented as a computer-readable storage medium, configured with a computer program, where the storage medium so configured causes a computer system to operate in a specific and predefined manner to perform the functions described herein.

The embodiments of the present invention that are described in this specification and the optional features and properties respectively mentioned in this regard should also be understood to be disclosed in all combinations with one another. In particular, in the present case, the description of a feature comprised by an embodiment - unless explicitly explained to the contrary - should also not be understood such that the feature is essential or indispensable for the function of the embodiment.

Fig. 1    shows an exemplary orientation sensor.

Fig. 2a    shows a patient's body with an implanted sensor with the patient's body in a first orientation.

Fig. 2b    shows a patient's body with an implanted sensor with the patient's body in a second orientation rotated by an angle $\alpha$ around the z-axis relative to the first orientation shown in Fig. 2a.

Fig. 3a    shows a flow chart for an exemplary method for determining a posture of a patient.

Fig. 3b      shows a schematic representation of a patient's body with an implanted acceleration sensor in an upright posture.

Fig. 3c      shows three schematic representations of a patient's body with an implanted three-dimensional sensor in an essentially horizontal posture.

Fig. 4       shows exemplary acceleration data represented as vector components along the three axes and of the acceleration sensor over time throughout a time period wherein the patient was standing, sitting, and lying down.

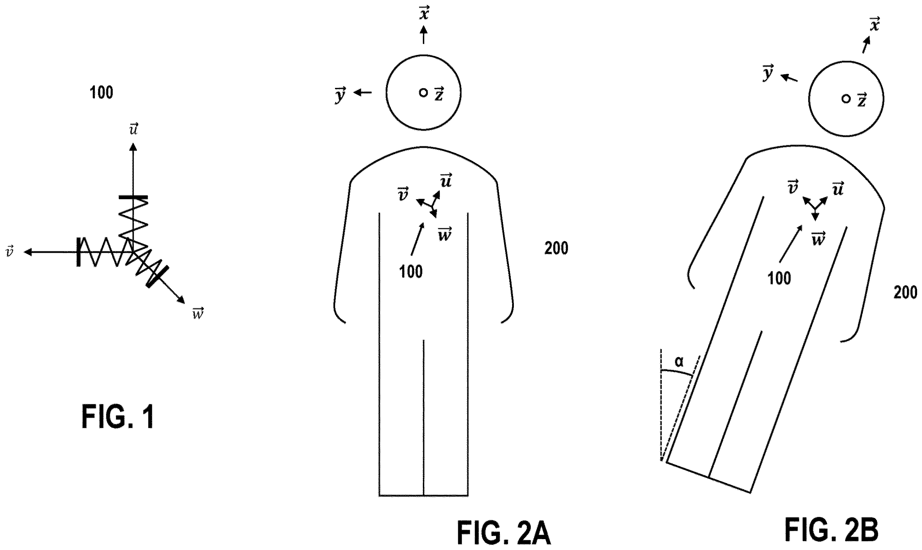

Fig. 1 shows an exemplary orientation sensor 100 with three axes $\vec{u}, \vec{v}, \vec{w}$. In the exemplary three-dimensional sensor 100 of Fig. 1, the three axes $\vec{u}, \vec{v}, \vec{w}$ are orthogonal such that each axes acquires the field strength of the external field along the direction of the respective axis. In the example of Fig. 1, the schematically shown springs shall show that the sensor 100 may sense the acceleration separately along the three axes $\vec{u}, \vec{v}, \vec{w}$ and/or provide the corresponding three-dimensional data to a further apparatus, e.g., a remote server (not shown).

The sensor 100 may, e.g., be an acceleration sensor measuring the gravitational field strength as described herein: Schematically, the sensor may act as if each of said springs are anchored to a common mount at one and to a mass m on the other end. According to Hooke's law, the spring force $\vec{F}$, the mass m is held at an equilibrium position against the gravitational force or the gravitational acceleration $\vec{g}$. Thus, the changes in the length of the spring may be proportional to the projections of the gravitational force on the respective axes $\vec{u}, \vec{v}, \vec{w}$ of the sensor 100: $\Delta s_{\vec{u}} = -\frac{m}{k} * \vec{u}^T * -\vec{g}$.
In total, this may yield:

$$-m * \vec{a} = \vec{F} = \vec{F}_{\vec{u}} + \vec{F}_{\vec{v}} + \vec{F}_{\vec{w}} = m * \left( (\vec{u}^T * -\vec{a}) * \vec{u} + (\vec{v}^T * -\vec{a}) * \vec{v} + (\vec{w}^T * -\vec{a}) * \vec{w} \right)$$

$$= -k * (\Delta s_{\vec{u}} * \vec{u} + \Delta s_{\vec{v}} * \vec{v} + \Delta s_{\vec{w}} * \vec{w})$$

The change in length of the springs $\Delta s_{\vec{u}}, \Delta s_{\vec{v}}, \Delta s_{\vec{w}}$ may be measured by the sensor 100 and the three-dimensional data may comprise the values $a_{\vec{u}}, a_{\vec{v}}, a_{\vec{w}}$, e.g.: $a_{\vec{u}} = -\frac{k}{m} * \Delta s_{\vec{u}} = \vec{u}^T * -\vec{a} = -\cos(\alpha) * |\vec{a}|$, wherein $\alpha$ may be the angle between $\vec{a}$ and $\vec{u}$. The same may apply analogously to $\vec{v}$ and $\vec{w}$.

Figs. 2a and 2b show exemplary embodiments, wherein the sensor 100 is an implantable sensor 100 to be implanted into a patient's body 200. However, the concept described in reference to said Figures may apply to any other body, e.g., as described herein, analogously.

Fig. 2a shows a patient's body 200 with an implanted orientation sensor 100 with the patient's body 200 in a first orientation. Said first orientation corresponds to the patient standing upright in the example of Fig. 2a. The sensor 100 may, e.g., be a sensor 100 according to the exemplary embodiment of Fig. 1. The axes $\vec{u}, \vec{v}, \vec{w}$ of the sensor 100 may be rotated relative to the patient axes $\vec{x}, \vec{y}, \vec{z}$, which, e.g., correspond to the direction of the head $\vec{x}$, the patent's right arm $\vec{y}$, and the patient's nose $\vec{z}$. In the example of Fig. 2a, the sensor (with its orientation expressed through the axes $\vec{u}, \vec{v}, \vec{w}$) is not aligned with the body (with its orientation expressed through the axes $\vec{x}, \vec{y}, \vec{z}$). It may, however, be aligned with them in other examples.

Fig. 2b shows a patient's body with an implanted orientation sensor 100 with the patient's body 200 in a second orientation rotated by an angle $\alpha$ around the z-axis relative to the first orientation shown in Fig. 2a. In comparison of Figs. 2a and 2b, one can see that obviously a rotation of the patient's body 200 brings along an equal rotation of the sensor 100, as they are fixedly connected to one another. Therefore, a change of orientation of the sensor 100 allows to be translated into a change of orientation of the body 200.

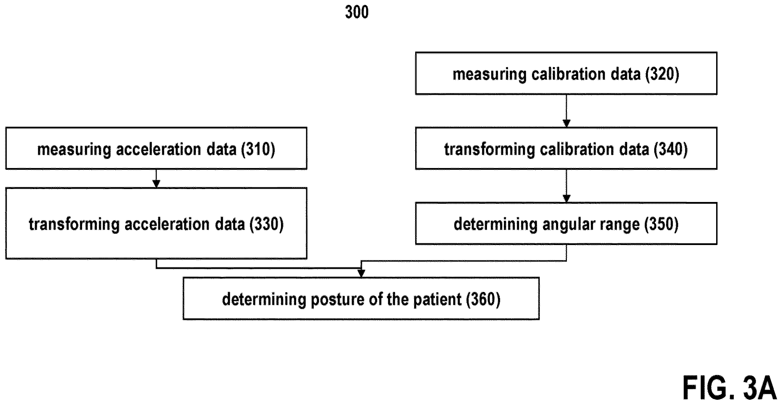

Fig. 3a shows a flow chart for an exemplary method 300 for determining a posture of a patient.

The method 300 comprises a step of measuring acceleration data 310 and subsequent a step of transforming said acceleration data 330, e.g., as described herein into one or more angular values.

Analogously, the method 300 of Fig. 3a comprises a step of measuring calibration data 320, a subsequent step of transforming said calibration data 340, e.g., as described herein into one or more angular calibration values. Based thereon, the method 300 comprises a step of determining at least one angular range 350 as described herein.

Lastly, the method 300 comprises a step of determining the posture of the patient 360 based on both, the at least one angular range and the angular value.

Fig. 3b shows a schematic representation of a patient's body with an implanted acceleration sensor in an upright posture.

Fig. 3c shows three schematic representations of a patient's body with an implanted acceleration sensor in an essentially horizontal posture. In detail, the left panel shows the patient lying on their left side, the central panel shows the patient in a supine posture lying on their back and the right panel shows the patient lying on their right side. This illustrates a typical range of motion of a patient, e.g., during a night.

Fig. 4 shows exemplary acceleration data 400 represented as vector components along the three axes and of the acceleration sensor over time throughout a time period wherein the patient was standing 401, 404, sitting 402, and lying down 403. In the example of Fig. 4, the w-axis essentially points in the direction of the patient's head.

The first panel shows the acceleration 400u au along the u-axis, the second panel shows the acceleration 400v along the v-axis, and the third panel shows the acceleration 400w along the w-axis. The fourth panel shows the total acceleration 400t which averages at around the value of 9.81 m/s$^2$ (earth's gravitational field strength).

In a first time period 401 and the fourth time period 404, the patient is standing/walking which results in the accelerations along the three axes of the sensor staying at constant levels 401u, 401v, 401w. This period is characterized by an elevated noise and standard deviation of the acceleration data 400.

Between the first time period 401 and the second time period 402, the patient sits down on a chair which results in increased noise of the acceleration data and variations of the measured accelerations, especially in the w-direction, indicating the posture change.

In the second time period 402 the patient is sitting on a chair which results in the accelerations along the three axes of the sensor staying at constant levels 401u, 401v, 401w. This period is characterized by low noise and low standard deviation of the acceleration data 400.

Between the second time period 402 and a second time period 403, the patient lies down which results in increased noise of the acceleration data and variations of the measured accelerations, especially in the w-direction, indicating the posture change.

In the third time period 403 the patient is lying which results in the accelerations along the three axes of the sensor staying at constant levels 401u, 401v, 401w. This period is characterized by low noise and low standard deviation of the acceleration data 400.

Between the third time period 403 and a fourth time period 404, the patient stands which results in increased noise of the acceleration data and variations of the measured accelerations, especially in the w-direction, indicating the posture change. In the fourth time period, the acceleration data along all axes 400u, 400v, 400w reach values that are similar to those in the first time period 401 (within some error margin).

It can be seen that the acceleration data 400w along the w-axis are most indicative for the described posture changes of the patient.

## Claims

1. Method (300) for determining a posture of a patient, the method comprising:

   obtaining at least one angular value representing acceleration data of the patient;
   obtaining posture data comprising at least one angular range representing at least one predetermined posture; and
   determining the posture of the patient (360) based at least partly on the at least one angular range and the angular value.

2. The method of claim 1, wherein the obtaining the at least one angular value comprises:

   measuring the acceleration data (310); and/or
   Transforming the acceleration data (330) into the at least one angular value, wherein
   optionally the transforming the acceleration data is based on a look-up table.

3. The method of claim 1 or 2, wherein the obtaining the posture data comprises:

obtaining, preferably measuring, calibration data (320) with the patient in the at least one predetermined posture; and,

transforming the calibration data (340) into at least one angular calibration value,

wherein optionally the transforming the calibration data is based on a look-up table.

4. The method of claim 3, wherein the calibration data comprise orthogonal data along at least two axes, preferably orthogonal data along three axes; and

wherein preferably the acceleration data and the calibration data comprise data along the same axes.

5. The method of claim 3 or 4, wherein obtaining the posture data comprises determining the at least one angular range (350) based at least partly on calibration data with the patient in the at least one predetermined posture.

6. The method of claim 5, wherein the determining the at least one angular range is based at least partly on a predetermined deviation, preferably between 0.1% and 10%, more preferably 0.5% to 5%, from the calibration data.

7. The method of any of claims 3 to 6, wherein the calibration data comprises at least:

first calibration data for a predetermined first posture of the patient and second calibration data for a predetermined second posture of the patient.

8. The method of claim 7, wherein the first and the second posture vary by 70° to 110°, preferably by 80° to 100°; wherein optionally the first posture comprises an upright posture of the patient and the second posture of the patient comprises a supine posture of the patient.

9. The method of any of claims 7 or 8, wherein the at least one angular range comprises at least: a first angular range based at least partly on the first calibration data, a second angular range based at least partly on the second calibration data, and preferably a global range representing a posture range comprising at least the first and the second posture.

10. The method of any of claims 7 to 9, further comprising obtaining, preferably for each axis, an average value of the first calibration data and the second calibration data; wherein the at least one angular range comprises at least one range around the average value.

11. The method of any of the previous claims, wherein the determining the posture of the patient is based at least partly on whether the calibration data lie within the at least one angular range.

12. The method of any of the previous claims, wherein the determining the posture of the patient comprises choosing a posture from a predetermined plurality of postures.

13. The method of any of the previous claims, wherein the acceleration data are obtained from an implantable sensor (100) implanted into a body (200) of the patient.

14. The method of any of the previous claims, further comprising a calibration step, the calibration step comprising:

measuring the calibration data with the patient in at least one predetermined posture.

15. An implantable medical device comprising:

means for obtaining an angular value representing acceleration data of the patient;

means for obtaining posture data comprising at least one angular range representing at least one predetermined posture; and

means for determining the posture of the patient based at least partly on the at least one angular range and the angular value.

FIG. 2B

FIG. 2A

FIG. 1

300

```
                                    ┌─────────────────────────────────┐
                                    │  measuring calibration data (320) │
                                    └─────────────────────────────────┘
                                                    │
                                                    ▼
┌─────────────────────────────────┐   ┌─────────────────────────────────┐
│ measuring acceleration data (310) │   │ transforming calibration data (340) │
└─────────────────────────────────┘   └─────────────────────────────────┘
                 │                                   │
                 ▼                                   ▼
┌─────────────────────────────────┐   ┌─────────────────────────────────┐
│ transforming acceleration data (330) │   │  determining angular range (350)  │
└─────────────────────────────────┘   └─────────────────────────────────┘
                 │                                   │
                 └───────────────┬───────────────────┘
                                 ▼
              ┌─────────────────────────────────────────┐
              │  determining posture of the patient (360) │
              └─────────────────────────────────────────┘
```

FIG. 3A

FIG. 3B

FIG. 3C

FIG. 4

Europäisches
Patentamt

European
Patent Office

Office européen
des brevets

# EUROPEAN SEARCH REPORT

**Application Number**

EP 24 19 6342

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/266025 A1 (HARELAND SCOTT A [US] ET AL) 25 August 2022 (2022-08-25) <br> * figures 6-9 * <br> * paragraphs [0080], [0100] - [0164] * <br> ----- | 1-15 | INV. <br> A61B5/11 <br> A61B5/00 |
| X | US 6 044 297 A (SHELDON TODD J [US] ET AL) 28 March 2000 (2000-03-28) <br> * figures 4-9 * <br> * column 14, line 39 - column 21, line 60 * <br> ----- | 1-15 | |
| X | US 2010/010380 A1 (PANKEN ERIC J [US] ET AL) 14 January 2010 (2010-01-14) <br> * figures 5-10 * <br> * paragraphs [0114] - [0196] * <br> ----- | 1-15 | |
| X | US 2007/118056 A1 (WANG HUA [US] ET AL) 24 May 2007 (2007-05-24) <br> * figures 1-6 * <br> * paragraphs [0036] - [0079] * <br> ----- | 1-15 | |

**TECHNICAL FIELDS SEARCHED (IPC)**

A61B

The present search report has been drawn up for all claims

| Place of search | Date of completion of the search | Examiner |
|---|---|---|
| The Hague | 28 January 2025 | Dhervé, Gwenaëlle |

CATEGORY OF CITED DOCUMENTS

X : particularly relevant if taken alone
Y : particularly relevant if combined with another
  document of the same category
A : technological background
O : non-written disclosure
P : intermediate document

T : theory or principle underlying the invention
E : earlier patent document, but published on, or
  after the filing date
D : document cited in the application
L : document cited for other reasons

& : member of the same patent family, corresponding
  document

EPO FORM 1503 03.82 (P04C01)

## ANNEX TO THE EUROPEAN SEARCH REPORT
## ON EUROPEAN PATENT APPLICATION NO.

EP 24 19 6342

This annex lists the patent family members relating to the patent documents cited in the above-mentioned European search report.
The members are as contained in the European Patent Office EDP file on
The European Patent Office is in no way liable for these particulars which are merely given for the purpose of information.

28-01-2025

| Patent document cited in search report | | | Publication date | Patent family member(s) | | Publication date |
|---|---|---|---|---|---|---|
| US 2022266025 | A1 | | 25-08-2022 | NONE | | |
| US 6044297 | A | | 28-03-2000 | DE 69910802 T2 | | 17-06-2004 |
| | | | | EP 1115350 A2 | | 18-07-2001 |
| | | | | US 6044297 A | | 28-03-2000 |
| | | | | WO 0018317 A2 | | 06-04-2000 |
| US 2010010380 | A1 | | 14-01-2010 | CN 102089032 A | | 08-06-2011 |
| | | | | CN 102137623 A | | 27-07-2011 |
| | | | | EP 2303122 A2 | | 06-04-2011 |
| | | | | EP 2307092 A2 | | 13-04-2011 |
| | | | | EP 2313004 A2 | | 27-04-2011 |
| | | | | EP 2313005 A2 | | 27-04-2011 |
| | | | | EP 2656787 A1 | | 30-10-2013 |
| | | | | US 2010010380 A1 | | 14-01-2010 |
| | | | | US 2010010381 A1 | | 14-01-2010 |
| | | | | US 2010010382 A1 | | 14-01-2010 |
| | | | | US 2010010384 A1 | | 14-01-2010 |
| | | | | US 2010010390 A1 | | 14-01-2010 |
| | | | | US 2010010583 A1 | | 14-01-2010 |
| | | | | US 2018071536 A1 | | 15-03-2018 |
| | | | | US 2023264029 A1 | | 24-08-2023 |
| | | | | US 2024115864 A1 | | 11-04-2024 |
| | | | | WO 2010005800 A2 | | 14-01-2010 |
| | | | | WO 2010005802 A2 | | 14-01-2010 |
| | | | | WO 2010005809 A2 | | 14-01-2010 |
| | | | | WO 2010005816 A2 | | 14-01-2010 |
| | | | | WO 2010005822 A2 | | 14-01-2010 |
| | | | | WO 2010005828 A2 | | 14-01-2010 |
| US 2007118056 | A1 | | 24-05-2007 | EP 1954192 A2 | | 13-08-2008 |
| | | | | JP 5260299 B2 | | 14-08-2013 |
| | | | | JP 2009515662 A | | 16-04-2009 |
| | | | | US 2007118056 A1 | | 24-05-2007 |
| | | | | WO 2007061746 A2 | | 31-05-2007 |

EPO FORM P0459

For more details about this annex : see Official Journal of the European Patent Office, No. 12/82